# EUROPEAN PATENT APPLICATION

(11) **EP 3 485 935 A1**
(43) Date of publication of application: **22.05.2019**
(21) Application number: 17202905.0
(22) Date of filing: 21.11.2017
(51) Int. Cl.: A61M 39/10, A61M 5/315, A61M 5/142

(54) **A ONE WAY COUPLING FOR AN INJECTION OR AN INFUSION DEVICE**

(71) Applicant: Tecpharma Licensing AG, 3401 Burgdorf (CH)
(72) Inventor: Hirschel, Jürg, 3007 Bern (CH); Hostettler, Patrick, 3415 Hasle (CH); Scheurer, Simon, 3006 Bern (CH)

(57) **Abstract**

A one-way coupling for an injection device or an infusion device, the one-way coupling comprising a first part being restricted for movement along and rotatable around a longitudinal axis, the first part having at least one pair of two engagement members which are positioned spaced apart from each other. At least one second part coaxially arranged with respect to the first part and having at least one pair of two corresponding engagement members configured for engaging the at least one pair of two engagement members of the first part, the second part being moveable with respect to the first part between a first and a second position. A third part coaxially arranged with respect to the first and second part, the third part being restricted for movement along the longitudinal axis and configured for engaging the second part such that the second part is rotationally secured with respect to the third part. Whereby rotation of the first part around the longitudinal axis in a first rotation direction relative to the third part brings one engagement member of the at least one pair of two engagement members in a mutual displacement engagement with one corresponding engagement member of the at least one pair of two corresponding engagement members, thereby displacing the second part from the first position to the second position or from the second position to the first position such that the second part moves back and forth between the first and second position. Whereby rotation of the first part around the longitudinal axis in a second rotation direction relative to the third part, the rotation of the first part being opposite to the first rotation direction, brings one engagement member of the at least one pair of two engagement members in a mutual locking engagement with one corresponding engagement member of the at least one pair of two corresponding engagement members such that the first, second and third part are coupled in a form-fit engagement for rotational movement in the second rotation direction and/or respective torque transmission.

## Description

### Background

Injection and infusion devices are used for the subcutaneous delivery of liquid medicaments to a patient. Such injection devices are often pen-shaped, having a long axis and are called injection pens. The injection pens comprise a housing, which can hold a dose setting and dose delivery mechanism. The medication is preferably present in a cartridge or in a prefilled syringe. A cartridge is normally attached to the housing of the injection pen using a cartridge holder. The user sets a dose of medication which is subsequently delivered from the cartridge. Such injection pens are used to deliver separate injections and not intended for continuous delivery of a medicament.

Infusion devices delivery the medication from the cartridge using a drive mechanism and a control mechanism that controls the advancement of a plunger present in the cartridge containing the medication. The medication is delivered to the patient via fluid path and an infusion set comprising a needle for subcutaneous delivery. With such infusion devices both continuous and temporary delivery profiles can be delivered to the patient.

A patch device is an example of an infusion device that is attachable to the skin of the patient. Such patch devices do not need tubing for connecting to the injection needle as the needle is directly contained in the patch device and inserted into the patient therefrom.

The injection and infusion devices comprise a dose setting mechanism, a delivery mechanism, a needle insertion and retraction mechanism or a needle shield protection system which is connected or connectable to the drive system. The drive system is fueled by a power source which supplies energy to the injection or infusion device for executing tasks such as medication delivery, establishing a connection between the fluid path and the cartridge, needle insertion, needle retraction, advancing and/or retracting a piston rod, signaling to the user that the medication is complete, signaling to the user that the device can be removed, powering a processor unit in the device or establishing a wireless connection for data transmission to an external and the like. The power source used in such injection or infusion devices can be selected from a wide variety of options such as, but not limiting to, a spring (compression, torsional spring, and leaf spring), an electromotor, a battery, pressurized gas or liquid-hydraulic systems and the like. In the injection and infusion devices, several operations need to be arranged in a certain sequence for a correct operation and transmission of power from the energy source to final medicament delivery, for example, by advancing a plug in a cartridge. For example for a patch infusion device, the needle must be inserted first, either using a steel needle (cannula) or a combination of a steel needle with a soft cannula; subsequently the steel needle must be retracted to leave the soft cannula in the subcutaneous tissue of the patient, followed by delivery of medication. Preferably, the needle, either soft of steel is retracted into the device before the patch device can be removed from the body. Alternatively, the needle is not retracted but a needle shield is extended from the body of the device to protect the needle tip and prevent needle stitching by the patient. All operating units need to be coupled and decoupled to the power source at a given moment in time to obtain a safe and reliable injection or infusion device therefore requiring coupling mechanisms, which can be either electrical or mechanical coupling mechanisms. The invention presented describes a mechanical coupling/decoupling mechanism.

The term "medicament" or "medication" includes any flow able medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

In WO02092153, a spring biased ratchet system is described which functions as a unidirectional coupling in an injection pen. A dose is set manually by rotating a scale drum of a dose setting mechanism in a first rotation direction. Delivery of a dose activates a unidirectional ratchet and a driver of a drive mechanism is rotated and the ratchet produces audible / tactile delivery clicks. The ratchet systems uses at least one spring which can get jammed and which adds complexity to the system and requires additional parts (springs) for the unidirectional couplings.
In EP0442516A1, a switchable freewheel coupling device is presented for an infusion device or an injection pump. The freewheel coupling comprises a rotatable shaft in a cylindrical housing and moveable jamming elements, such as balls, located between the housing and the shaft. The jamming elements are biased by a spring such that relative rotation between the shaft in one rotation direction ensures that the housing is co-rotated with the shaft due to the blocking jamming elements, in the opposite rotation direction the rotation of the shaft is not transmitted to the cylindrical housing. The operating principle of the freewheel requires biasing members for each jamming element, in total at least 4 parts.
One way ball clutches are presented in US5743889 for an injection pen. During dose setting a first ball clutch prevents axial movement of a piston rod, and the bias of this first ball clutch is released during dose delivery where a second biased ball clutch is engaged to transfer a linear movement of the dose setting mechanism in a linear movement of the piston rod. The one-way ball clutches presented are biased by a spring, reducing the reliability and adding parts to the system.
The one-way clutch systems of the prior art based on ratchets inevitably produce noise which is in some applications is not desired, also the ratchet systems use springs which adds parts to the system and, depending on the spring force, increases the frictional losses of the ratchet. The rotational and axial one way ball clutches require biasing members, thereby adding parts to the system. Additionally, the springs themselves can get blocked, thereby bypassing the one way clutch reducing the reliability of the one-way clutch.

It is an object of the present invention to provide a one-way coupling mechanism for an injection device or an infusion device which is simple in construction, reliable in use and limits the number parts required. The one-way coupling acts as a freewheel, and preferably transmits the rotational torque in one rotation direction from a first part to a second and/or third part without using mechanical springs and using a minimum amount of parts.

This object is solved by the independent claim of the invention in that a first part that is rotatable around a longitudinal axis in a first direction and urges a second part to oscillate back and forth between a first and a second position, preferably without rotation of the second part around the longitudinal axis. The second part is driven by engagement members and corresponding engagement members present on the first and second part, e.g. driven between the two positions without using a biasing force of a spring member. Rotation of the first part in a first rotation direction ensures that an engagement member of the first part displaces a corresponding engagement member of the second part such that the second part is moved from the first to the second position or vice versa. Rotation of the first part in the second direction opposite to the first direction ensures that the engagement member of the first part enters into a locking engagement with a corresponding engagement member of the second part thereby slaving the second part such that the first and second part co-rotate in the second rotation direction. Preferably the second part is rotationally secured to a third part such that the third part co-rotates with the first and second part in the second rotation direction. The engagement members are preferably present as at least one pair of opposing engagement members and the corresponding engagement members are preferably present as at least one pair of corresponding engagement members. During the oscillating movement of the second part between the first and second position, a pair of engagement members of the first part repeatedly engages and disengages from a pair of corresponding engagement members of the second part.
In another aspect of the invention an injection device, infusion device or patch injector is described which comprises the one-way coupling.

### General description

Disclosed is a one-way coupling, or a so called freewheel coupling or one-way clutch for an injection device or an infusion device. The injection device or infusion device may be a patch device such as a patch injector or a bolus injector. The device comprises a housing for giving structural support to the different parts of the device such as a drive mechanism or an insertion mechanism, preferably the housing is attached or attachable to the skin of the patient, preferably using an adhesive layer.

The one-way coupling comprises a first part defining a longitudinal axis and the first part is restricted for movement along and rotatable around the longitudinal axis. The first part preferably has a cylindrical shape and the longitudinal axis defined preferably equals the longitudinal axis of the cylinder shape. The first part has at least one pair of two engagement members which are positioned spaced apart from each other. Spaced apart is defined here as that there is a distance between the two engagement members of each pair of engagement members. The distance between the two engagement members is preferably identical for each pair of engagement members. The one-way coupling comprises at least one second part that is coaxially arranged with respect to the first part. The second part has at least one pair of two corresponding engagement members configured for engaging the at least one pair of two engagement members of the first part whereby the second part is moveable with respect to the first part between a first and a second position. A pair of engagement members of the first part can repeatedly engage or disengage from a pair of corresponding engagement members of the second part. Preferably one second part is used, but it may be advantageous to use more than one second member in order to reduce the play between the first part and the second part before the engagement members and corresponding engagement members interact with each other.

The one-way coupling furthermore comprises a third part that is preferably coaxially arranged with respect to the first and second part. Coaxial is defined as having a common rotational axis, e.g. the third part and the first and second part have a common rotational axis. The third part is preferably restricted for movement along the longitudinal axis and configured for engaging the second part such that the second part is rotationally secured with respect to the third part, e.g. the second and third part preferably rotate together. Preferably, the second part and the third part are axially moveable with respect to each other or the second part can tilt with respect to the third part while still being in a rotational locked engagement relative to another.

The one-way coupling is characterized by that rotation of the first part around the longitudinal axis in a first rotation direction relative to the third part brings one engagement member of the at least one pair of two engagement members in a mutual displacement engagement with one corresponding engagement member of the at least one pair of two corresponding engagement members, thereby displacing the second part from the first position to the second position or from the second position to the first position such that the second part moves back and forth between the first and second position. When the one engagement member of the pair of engagement members interacts with the corresponding engagement member of the pair of corresponding engagement members then the second part is moved from one position to the other position thereby bringing the other engagement member of the pair of engagement members in interaction with the other corresponding engagement member of the pair of corresponding engagement members. The mutual displacement engagement is preferably established by sloped or curved surfaces of, or on the engagement members and corresponding engagement members that can contact one another in the first or second position and when the engagement members and corresponding engagement members are rotated relative to another, the contact surfaces slide over each other whereby the engagement member and corresponding engagement member move apart, preferably along the axis of the relative rotation.

Rotation of the first part around the longitudinal axis in a second rotation direction relative to the third part, the rotation of the first part being opposite to the first rotation direction, brings one engagement member of the at least one pair of two engagement members in a mutual locking engagement with one corresponding engagement member of the at least one pair of two corresponding engagement members. The mutual locking engagement is preferably established by sloped or curved surfaces of, or on the engagement member and corresponding engagement member that can contact each other. Relative movement, preferably relative rotational movement, of the engagement member and corresponding engagement member ensures that the surfaces slide over each other until a form-fit engagement between the engagement member and corresponding engagement member prevents further relative movement. Preferably, the engagement member and corresponding engagement approach each other when the complementary sloped or curved surfaces slide over each other, the approaching is preferably along the axis of relative rotation, until a form-fit engagement between the engagement member and corresponding engagement member prevents relative axial and/or rotational movement between the engagement member and the corresponding engagement member. When one of the engagement member of the at least one pair of engagement members engages one of the corresponding engagement member of the at least one pair of corresponding engagement members in a mutual locking engagement, then the first, second and third part are coupled in a form-fit engagement for rotational movement in the second rotation direction and/or respective torque transmission. The locking engagement ensures that two complementary shapes or surfaces of the engagement member and corresponding engagement member are interlocked by rotation of the first part in the second rotation direction. The rotation or torque transmission is preferably with respect to a housing or base of the injection or infusion device. The mutual locking engagement ensures that the second part preferably is locked in either the first position or the second position.

The movement back and forth between the first and second position of the second part when the first part rotates in the first rotation direction produces little or no noise and the axial displacement is done without the use of a separate resilient member thereby reducing the complexity of the coupling.

The second part is locked either in the first position or the second position when the first part rotates in the second rotation direction, e.g. rotation in the second rotation direction ensures that the engagement members and corresponding engagement members always engage in one of the two positions which enhances the reliability of the system together with the absence of a separate resilient member.

The two engagement members of each pair of the at least one pair of two engagement members of the first part are spaced apart and oppositely directed and parallel or transversally aligned with respect to the longitudinal axis. Preferably, the two engagement members of each pair of engagement members are spaced apart parallel to the longitudinal axis defined by the first part. When more than one pair of engagement members is used, then all pairs are preferably spaced apart at the same distance. If more than one pair of engagement members is used, then each pair is preferably circumferentially arranged parallel to and around the longitudinal axis.

Alternatively, the at least one pair of engagement members is spaced apart and transversally or perpendicular oriented to the longitudinal axis defined by the first part. If more than one pair of engagement members is used, then each pair has preferably the same distance between the engagement members. Preferably the pairs of transversally oriented engagement members are positioned at equal angles with respect to each other around the longitudinal axis when more than one pair of engagement members is used.

The corresponding engagement members are arranged as pairs on the second part. The corresponding engagement members of each pair of two corresponding engagement members of the second part are oppositely directed and arranged between the at least one pair of two engagement of the first part. The corresponding engagement members of the second part are preferably oriented parallel to the engagement members of the first part. For example when the pairs of engagement members of the first part are oriented parallel to the longitudinal axis, then the pairs of corresponding engagement members are also oriented parallel to the longitudinal axis. If the engagement members are transversally oriented, then also the corresponding engagement members are transversally oriented.

The first and second position of the second part in the one-way coupling is defined by the position of the two engagement members of the at least one pair of two engagement members of the first part. The movement of the second part is restricted by the position of the engagement members of the first part that are spaced apart a distance. The distance the second part travels between the first and second position is thus restricted by the position of the engagement members of the at least one pair of engagement members of the first part. Preferably, each engagement member of the pair of engagement members is each coupled to, or part of a base and therewith the position of the second part can also be defined by the bases of a pair of engagement member.

The second part moves back and forth between the first and second position and this movement is parallel or, perpendicular to the longitudinal axis as defined by the first part. Alternatively, the second part is tilted with respect to the longitudinal axis during movement from the first position to the second position or performs a combination of a tilting movement and an axial movement. During the movement back and forth of the second part, preferably one pair of engagement members of the first part repeatedly engages and disengages from one pair of corresponding engagement members of the second part.

The one-way coupling wherein the first, second and third part are sleeve shaped or shaft shaped. The sleeve shaped first, second and third part are preferably coaxially oriented with respect to each other. Preferably, the second part is located between the first and third part, as an alternative the second and third part are surrounded by the first part.

The second part is axially moveable with respect to the third part. Preferably the second part is splined with respect to the third part, alternatively there is a gearing engagement between the second and third part.

The second part is preferably rotationally secured with respect to the third part.

The second part preferably can axially move with respect to the third part or tilt with respect to the third part.

The one-way coupling wherein the second part moves back and forth between the first and second position without rotation around the longitudinal axis when the first part rotates in the first rotation direction. The third part, preferably rotationally locked to the second part is preferably non-rotating when the first part rotates in the first rotation direction. The one way coupling wherein the first, second and third part rotate in the second rotation direction when the first part is rotated in the second rotation direction.

The one way coupling wherein the second part has an engagement means for rotationally locking to the third part, preferably designed as a radial protrusion on the second part that engages a radial recession on the third part, or designed as a radial recession on the second part that engages a radial protrusion on the third part. The engagement means allows for relative axial or pivoting movement between the second and third part. The radial protrusion or recession on the second part is preferably oriented parallel or tilted with respect to the longitudinal axis as defined by the first part.

The one way coupling wherein the second part is rotationally engaged in a friction fit engagement with respect to the housing, either directly for example by a press fit engagement to the housing of the device (or base of the device) or indirectly via an additional part such as an O-ring or the third part. Preferably, the second part is in a friction with engagement with the housing via the third part. The second and third part are preferably rotationally locked with respect to each other. The third part is in a friction fit engagement with the housing, for example via a press fit or via an O-ring. The third part is preferably prevented from axial movement with respect to the housing but a initial frictional force needs to be overcome before the third part, and preferably also the second part, start rotating in the second rotation direction. Once a threshold torque is generated to the third part via the second part due to rotation of the first part in the second rotation direction, the first second and third part co-rotate in the second rotation direction. The friction fit engagement between the third part and the housing (or a base) ensures that a water tight and sterile barrier is formed between the third part and the housing which ensures that different compartment within the housing can be separated from each other and also separately be sterilized from another. The one-way coupling ensures that mechanical members or electrical switches across such sterile barriers can be mechanically coupled and decoupled from another.

The one-way coupling according to any of the previous claims wherein the first part is axially supported by a bearing engagement between the first part and the third part. The first part of the one way coupling is surrounded preferably by the second and the third part. The first part is preferably axially supported by the third part and/or the second part. For example, the first part is shaped as a sleeve attached to a rotatable shaft on one end whereas the opposite end is axially supported by a bearing or protrusion in the third part engaging the other end of the sleeve of the first part. The third part is guided in the housing and thus forces perpendicular to the rotational axis of the shaft are guided from the first part via the bearing of the third part to the housing.

Preferably, the second part is a sleeve having a longitudinal opening to form a C-arc that can be clipped onto the first part or wherein the first part is constructed as two sub-parts that can be assembled together after insertion of a second part that is shaped as a continuous sleeve. In this configuration, the second part surrounds the first part and for ease of assembly either the second part is clipped onto the first part and/or the first part is made from two sub parts. As an alternative, the second part is molded, for example injection molded around the second part or the first and second part are made using 3D printing techniques such that both parts are produced in a single production step.

The one-way coupling wherein the at least one pair of two engagement members are circumferentially arranged on the first part around the longitudinal axis and the at least one pair of two corresponding engagement members are circumferentially arranged on the second part. Circumferentially arranged means that a line connecting the pair of engagement members or pair corresponding engagement members can be oriented parallel to the longitudinal axis defined by the first part, but the ends of the connecting lines are disposed on a circle having the longitudinal axis as a center. The two engagement members of the at least one pair of two engagement members of the first part are positioned face-to-face to each other and the two corresponding engagement members of the at least one pair of two corresponding engagement members are positioned off-set to each other.

Alternatively, the engagement members of the at least one pair of two engagement members of the first part are positioned off set to each other and the two corresponding engagement members of the at least one pair of two corresponding engagement members of the second part are positioned face-to-face to each other. Off-set to each other means that the line connecting the pair of engagement members (or corresponding engagement members) is at an angle with respect to the longitudinal axis. Combining the face-to-face arrangement with an off-set arrangement of the pairs enables a relative zig-zag movement of the pairs of corresponding engagement members with respect to the pairs of engagement members.

The number of pairs of engagement members and corresponding engagement members defines the number of back and forth shifts of the second part when the first part rotates in the first rotation direction and defines the play before the first and second part interlock when the first part rotates in the second rotation direction. For example 4 pairs of two engagement members can be positioned at an equal angle around and parallel to the longitudinal axis at 0°, 90°, 180°, 270° and be combined with one pair of corresponding engagement members on the second part. This results in four back and forward movements of the second part per revolution of the first part and a maximum rotational play until one of the engagement members engages one of the corresponding engagement members. By increasing the number of pairs of engagement members and corresponding engagement members, the rotational play until an engagement member engages a corresponding engagement member either in a displacement or locking engagement is reduced. Additionally, by increasing the pairs of engagement members and corresponding engagement members the rotational torque that is transmitted from the first part to the second part via the locking engagement is distributed over a number of engagements, thereby reducing the load transferred per engagement.

Alternatively, the rotational play before the first part engages the second part in a mutual locking engagement and the first and second part co-rotate in the second rotation direction, can be tuned by using a second part that comprises more than one part. Each part of the second part comprises at least one pair of corresponding engagement members. The first part comprises more than one set of pairs of engagement members that each can interact with the corresponding engagement members of the parts forming the second part.

The engagement members and corresponding engagement members of the one-way coupling are preferably shaped as teeth. Such teeth can by symmetric teeth or asymmetric teeth and protrude from a base. Symmetric teeth have two sloped flanks or surfaces with an equal angle to the base. An example of asymmetric teeth are saw teeth having one steep surface and one shallow surface. The steep surface or flank of a tooth has a higher angle with respect to the base compared to the shallow surface of that same tooth.
The saw teeth are defined here as having a saw cut surface and an undercut surface. The undercut surface is the surface having a flank with a steep slope with respect to the base whereas the saw cut surface has a shallow slope with respect to the base.

The mutual displacement engagement and/or the mutual locking engagement is caused by sloped surfaces of the teeth of the engagement members and the corresponding engagement members sliding along each other. A sloped surface or flank of an engagement member contacts with, and slides over a sloped surface of a corresponding engagement member thereby causing the engagement member and the corresponding engagement member to move towards each other (locking engagement) or thereby causing a separation from each other (displacement engagement). The relative movement of sloped surfaces sliding along each other is preferably perpendicular to the longitudinal axis whereas the locking or displacement engagement is preferably parallel to the longitudinal axis. The relative movement of sloped surfaces sliding along each other is preferably caused by a relative rotational movement of the first and the second part.

The engagement members of the first part protrude from a base present on the first part. Preferably each engagement member of the two engagement members protrudes from a base, e.g. the first part comprises at least two bases having each one of the two engagement members of the pair. The two bases are preferably spaced apart and the two engagement members of a pair are oppositely oriented, preferably towards each other. For example if the engagement members are tooth shaped, then the two engagement members point towards each other (face each other) or, as an alternative, the two teeth can point in opposite directions. The second part comprises at least one pair of two corresponding engagement members which each protrude preferably from two bases present on the second part. In the example presented before where the corresponding engagement members are tooth shaped, the two teeth of a pair of teeth on the second part can point in opposite directions, which can be combined with a pair of facing teeth on the first part or, vice versa, a pair of teeth facing each other on the second part preferably engage with a pair of opposing teeth on the first part.

The pair of engagement members of the first part and corresponding engagement members preferably protrude from a base and are oriented in a direction that is parallel to the longitudinal axis of the first part. As an alternative, the pair of engagement members and corresponding engagement members protrude from a base and are oriented transversally to the longitudinal axis. In the latter case, the pair of engagement members and pair of corresponding engagement members are radially arranged with respect to the longitudinal axis defined by the first part. Preferably the radially arranged pairs of engagement members of the first part protrude outward from the outer surface of a first cylinder comprised in the first part and which are configured to engage at least one pair of corresponding engagement members protruding inwards from an inner surface of a second cylinder surrounding the first cylinder; the second cylinder defining the second part. The outer surface of the first cylinder and the inner surface of the second cylinder thereby defining the base for the engagement members and corresponding engagement members.

Preferably, the base for the teeth is formed as radially extending flanges present on the first part or on the second part. The second part surrounding the first part implies that either the first part comprises flanges radially extending outwards and/or the second part comprises radially inwardly directed flanges to support the engagement members and/or the corresponding engagement members. As an alternative, the first part surrounds the second part requiring radially inwardly directed flanges on the first part and radially outwardly directed flanges on the second part. Preferably, the second part is present in a recessed section of the first part or between a proximal and distal flange present on the first part, wherein the base for the engagement members is formed by ridges of the recessed section of the first part, or by the flanges present on the first part. The base for the corresponding engagement members preferably is the top and bottom rim of the sleeve shaped second part. The radially inwardly and outwardly extending flanges as a base for the engagement members is preferably combined with engagement members and corresponding engagement members that are oriented parallel to the longitudinal axis defined by the first part.

The one way coupling wherein the teething of the engagement members and corresponding engagement members comprises saw teeth, and wherein the locking engagement is defined by the mutual interaction of the undercut surfaces of a saw tooth from an engagement member with an undercut surface of a saw tooth from a corresponding engagement member causing a form fit arrangement between the engagement member and the corresponding engagement member when the two undercut surfaces slide along each other. The locking engagement is not a permanent locking engagement but a releasable locking engagement. The tip of a saw tooth of an engagement members slides along the undercut surface of a corresponding engagement member and this relative movement between the sloped surfaces ensures that the engagement member and the corresponding engagement member axially approach each other. The relative movement between the engagement member and corresponding engagement member is halted when the tip of the engagement member reaches the base of the undercut surface of the corresponding engagement member defining the form fit arrangement. The form fit arrangement equally applies when a tip of a saw tooth of a corresponding engagement member slides over an undercut surface of a saw tooth of an engagement member and a form fit arrangement is defined by the tip of the corresponding engagement member abutting the base of the undercut surface of the saw tooth of the engagement member.

The locking engagement ensures that two complementary shapes or surfaces of the engagement member and corresponding engagement member are interlocked by rotation of the first part in the second rotation direction. The complementary shapes or surfaces can be hooks, teeth, asymmetric teeth such as saw teeth mating convex-concave shapes, curved surfaces, sloped surfaces and the like. Optionally the shapes or surfaces are attached to a resilient arm or member protruding from the first and/or second part.

The locking engagement ensures that the second part is releasable locked in either the first or the second position. The first and second position are preferably defined along the longitudinal axis or perpendicular to the longitudinal axis defined by the first part. The locking engagement can be released by rotating of the first part in the first rotating direction whereby the form-fit engagement is released and the first and second part can engage in a mutual displacement engagement.

The displacement engagement is defined by the mutual interaction of a saw-cut surface from an engagement member with a saw cut surface from a corresponding engagement member causing a gearing arrangement between the engagement member and the corresponding engagement member when the two saw cut surfaces slide along each other causing relative axial displacement of the engagement member and corresponding engagement member.

Either the first part comprising the engagement members or the second part comprising the corresponding engagement members is axially fixed with respect to the longitudinal axis thus ensuring that there is a relative movement between the first and second part during the displacement engagement, where preferably the second part is moved from the first to the second position or from the second to the first position. The movement is preferably parallel or perpendicular to the longitudinal axis defined by the first part.

The first and second part are in a mutual displacement engagement when the first part is rotated in the first rotation direction and the second part shifts back and forth from the first to the second position. An initial relative rotational play exists before the first part and the second part are in a mutual displacement engagement which depends on the number and position of the engagement members and corresponding engagement members. Per revolution (360°) of the first part, the second part performs at least one back and forth movement. The one-way coupling is designed such that the rotation direction of the first part can be repeatedly switched from the first rotation direction to the second rotation direction. The mutual locking engagement is repeatedly released when switching from the second to the first rotation direction such that the first and second part are set in a mutual displacement engagement. Switching the rotation direction back to the first rotation direction bring the first and second part in the mutual locking engagement.

An Injection or infusion device comprising the one way coupling embodiments described above, the injection or infusion device is configured for coupling and decoupling the rotational movement of an electromotor or a torsional spring to a drive mechanism or a needle insertion mechanism by switching the rotation direction of the first part from the first direction to the second rotation direction. Alternatively the rotation direction of the first part relative to the third part is switched from the second rotation direction to the first rotation direction. In the first rotation direction the first part rotates whereas, preferably, the second and third part do not rotate whereas in the second rotation direction the first second and third part rotate. The needle insertion and/or retraction mechanism is preferably directly or indirectly coupled to the third part. The electromotor or the torsional spring is either directly or indirectly coupled to the first part such that switching the rotation of the electromotor results in coupling or decoupling of the needle insertion system via the third part.

The first, second and third part are preferably made from a polymeric material which can be selected from
Polyoxymethylene (POM), polybutyleneterphthalate (PBT), Polyethyleneterphtahlate (PET), Polyamides (PA), Polycarbonate (PC), Acylonitrile-butadiene-styrene (ABS), Polyphenylsulfone (PPSU), Polysulfone (PSU), Polyether-ether-ketone (PEEK), Polymethylene-methacrylate (PMMA), Polystyrene (PS), Cycloolefinic copolymer (COC), Polyolefines such as polyethelenes (PE, HDPE, UHMWPE) or polypropylene (PP), Liquid crystalline polymers (LCP,Vectra). Thermosetting polymers such as epoxy or polyurethane resins (PUR) may be used as well. Alternatively metals or ceramics may be used for the first, second or third material such as stainless steel or aluminiumoxide. The polymeric materials can be further strengthened by adding glass or carbon fibers to the material. Friction reduction may be beneficial for reducing noise generation and energy losses by the one-way clutch during use. An option to reduce the friction is to add a lubricant to the one-way clutch, for example the contacting surfaces can be coated with a friction reducing layer comprising graphite, fluoropolymers, or silicone oil/grease. Alternatively, the friction reduction can be achieved by incorporation of the friction reducing compounds in the polymeric material. For example, the first, second and third parts are made from polycarbonate, polybutyleneterphthalate (PBT) or polyoxymethylene (POM) wherein one or both of the materials is optimized to reduce friction by incorporation of a silicone oil, graphite, or a fluorocompound.

### Legends to Figures

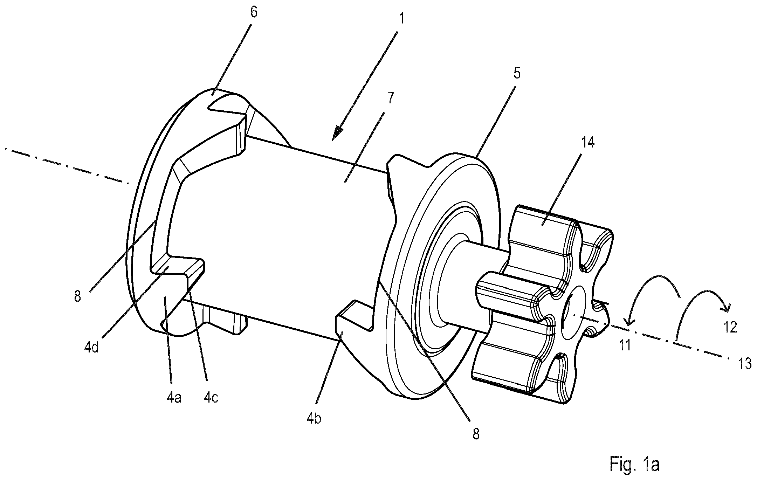

Figure 1a: First part of the one-way coupling according to the first embodiment.

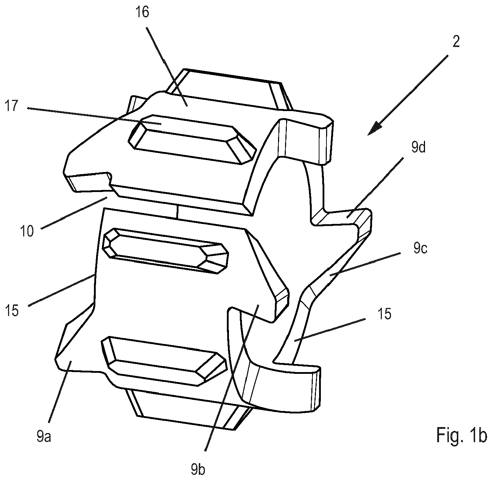

Figure 1b: Second part of the one-way coupling according to the first embodiment.

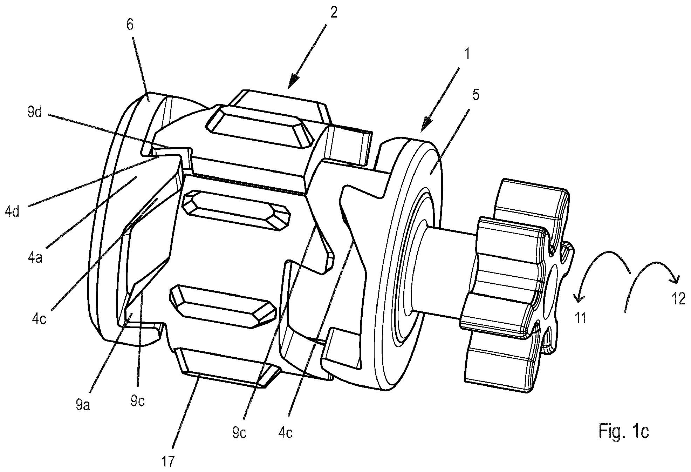

Figure 1c: First part and second part assembled of the one-way coupling according to the first embodiment.

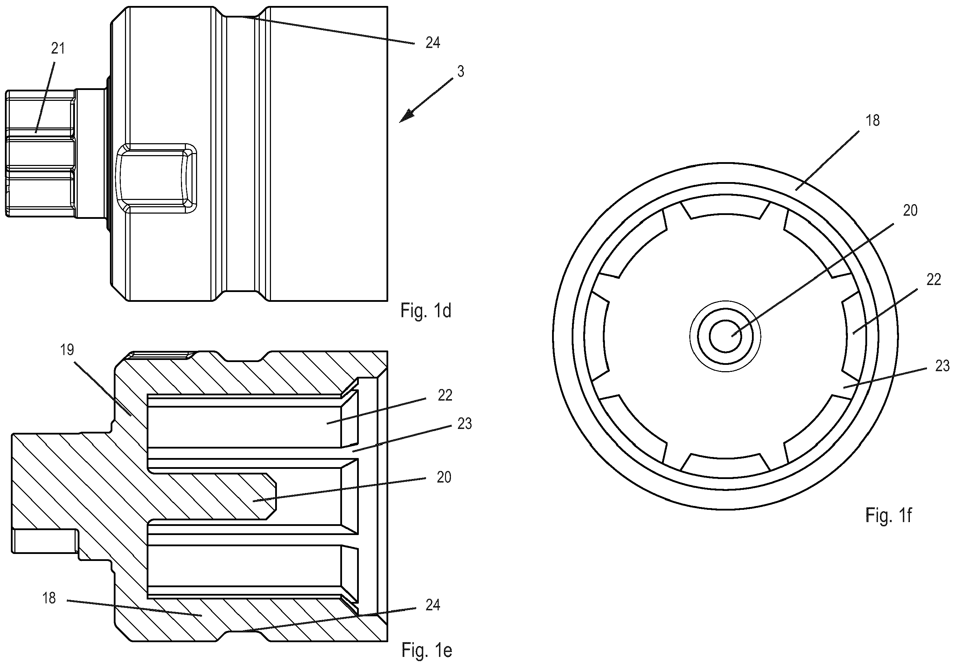

Figure 1d: Third part of the one-way coupling according to the first embodiment.
Figure 1e: Longitudinal section of third part of the one-way coupling according to the first embodiment.
Figure 1f: Third part of the one-way coupling according to the first embodiment.

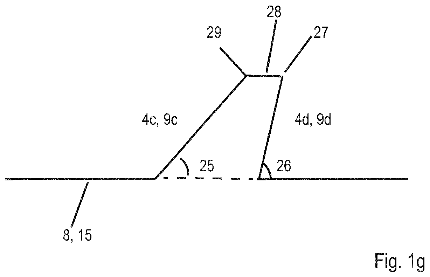

Figure 1g: Schematic drawing showing saw tooth definition.
Figure 1 h: Schematic drawing showing the mutual locking engagement.
Figure 1i: Schematic drawing showing the mutual displacement engagement.
Figure 2a: Longitudinal section of assembled one-way coupling according to the first embodiment.
Figure 2b: Assembled one-way coupling according to the first embodiment.
Figure 3a: First part of the one-way coupling according to the second embodiment.
Figure 3b: First part of the one-way coupling according to the second embodiment.
Figure 3c: Second part of the one-way coupling according to the second embodiment.
Figure 4a: First part and second part assembled of the one-way coupling according to the second embodiment.
Figure 4b: Cross section of Figure 4a, second part in first position.
Figure 5a: Cross section of one way coupling according to the second embodiment, second part in second position.
Figure 5b: One way coupling according to the second embodiment.
Figure 6a: One way coupling according to the third embodiment, first and second part assembled.
Figure 6b: One way coupling according to the third embodiment, first and second part assembled.
Figure 7: Third part of the one way coupling according to the third embodiment.
Figure 8a: One way coupling according to the third embodiment, second part in a first position.

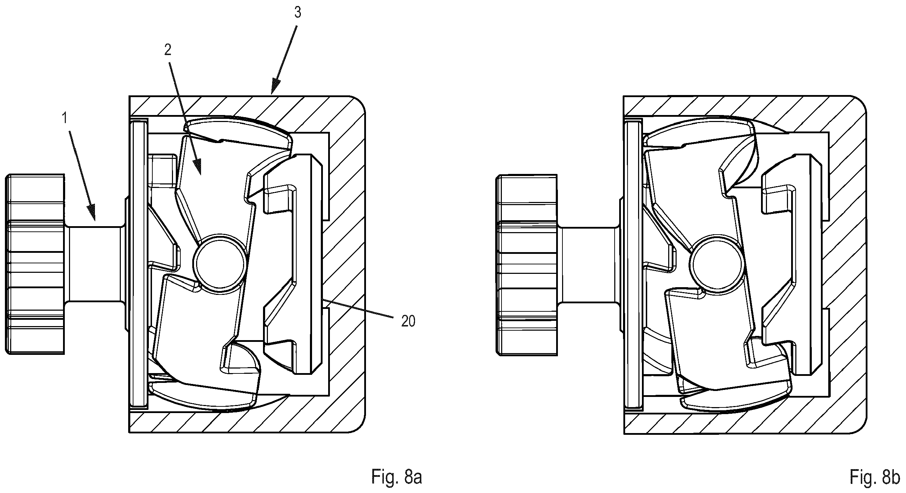

Figure 8a: One way coupling according to the third embodiment, second part in a second position.

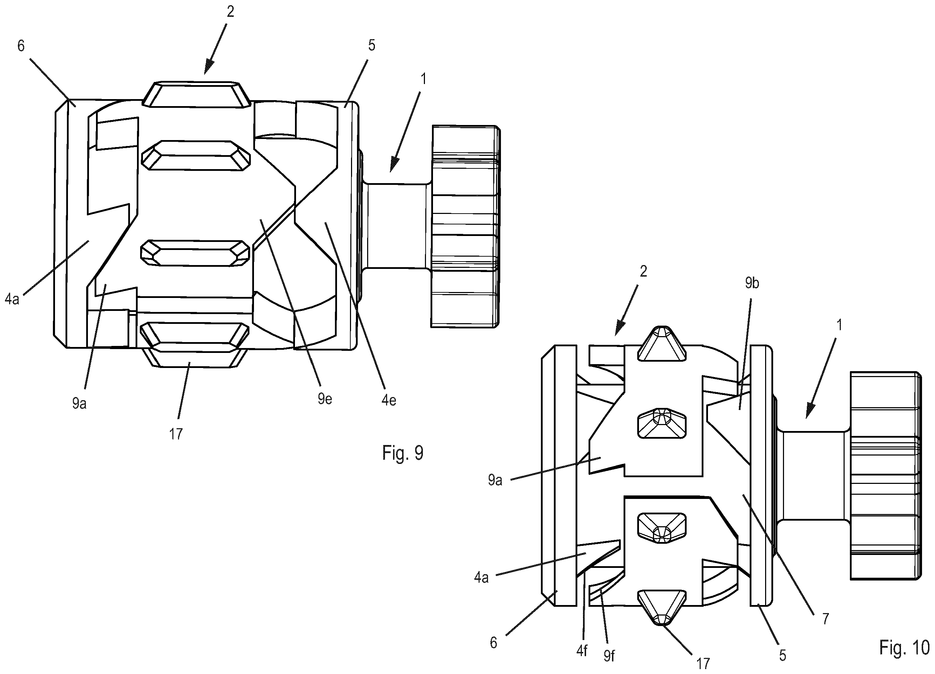

Figure 9: One way coupling according to a fourth embodiment, first and second part assembled.
Figure 10: One way coupling according to a fifth embodiment. First and second part assemblked.

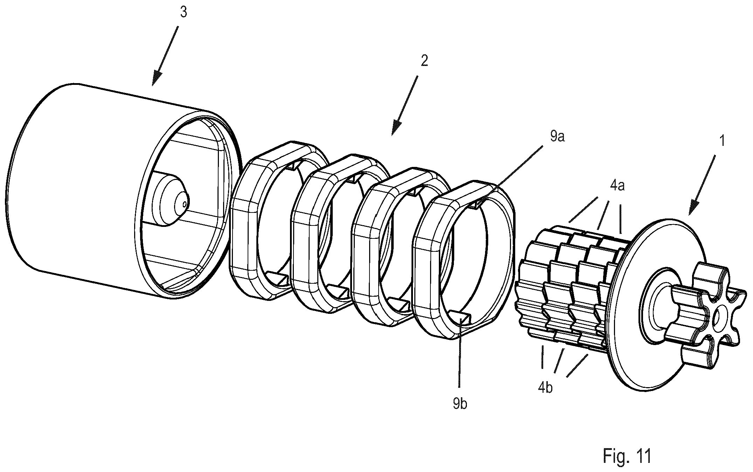

Figure 11: One way coupling according to a sixth embodiment.

### Detailed description

The first embodiment of the invention is shown in Figures 1a to 1 f. In Figure 1a, a first part (1) is presented having a cylindrical body or sleeve (7) that can be rotated around the axis (13) of the cylinder in a first direction (11) and in a second direction (12) which is opposite to the first direction. In this embodiment, the first part 1 comprises a gear wheel (14) connected to and preferably designed for rotating the first part, for example via a second gear wheel (not shown). When viewed from the gear wheel (14) towards the end of the first part (1), the first rotation direction (11) is the counterclockwise direction whereas the second rotation direction (12) is the clockwise rotation direction. In this embodiment. The first part comprises a first flange (5) and second flange (6) that radially extend outward from the sleeve (7). On each flange (5, 6) a pair of engagement members (4a, 4b) is positioned, preferably circumferentially around the longitudinal axis (13). The engagement members of the pair (4a, 4b) point towards each other and are each shaped as an asymmetric saw tooth. The engagement members protrude from a base (8) which in this embodiment is formed by the surface of the radially extending flanges (5, 6). Each engagement member (4a, 4b) comprises a saw cut surface (4c) and an undercut surface (4d) that start at, and extend from the base (8). The undercut surface (4d) has a higher angle with respect to the base (8) then the saw cut surface (4c). In other words the undercut surface is steeper with respect to the base and the saw cut surface has a shallower slope. In Figure 1g, a schematic drawing of a saw tooth is shown explaining the definition of the undercut surface and the saw cut surface and what angle is meant when referring to the base (8). In Figure 1a, four pairs of two engagement members (4a, 4b) are shown that are evenly distributed at the flanges (5, 6) of the first part (1). The pairs of engagement members (4a, 4b) all face each other such that a line connecting the pairs of engagement members is parallel to the longitudinal axis (13). The four pairs are evenly distributed around the circumference of the flanges (5, 6) and interspaced at an angle of 90° with respect to a neighboring pair of engagement members.

Figure 1b, the second part (2) is presented as sleeve having an opening (10) to form a C-shaped sleeve. The second part (2) comprises four pairs of two corresponding engagement members (9a,9b) shaped as asymmetric teeth that are oppositely oriented and protrude from a second base (15) which is the top and bottom surface of the sleeve. Each corresponding engagement member of a pair (9a, 9b) is off-set to the other corresponding engagement member of the pair (9a, 9b), e.g., positioned at a different angle on the circumference of the second base (15). This implies that a line connecting the corresponding engagement members (9a, 9b) is angulated with respect to the longitudinal axis of the second part (2), which for this embodiment equals the longitudinal axis (13) of the first part (1). Each engagement member of each pair of engagement members (4a,4b) is positioned at an equal angle on the flanges of the sleeve shaped first part (1), whereas each corresponding engagement member (9a,9b) of each pair of corresponding engagement members is positioned at a different angle on the base areas of the second part (2). This situation can also be reversed, e.g. each corresponding engagement member is paired with another corresponding engagement member on the second part at an equal angle (facing directly opposite to another) and each pair of engagement members (4a, 4b) is positioned off-set to each other on the first part (1).

The C-shaped sleeve is preferably made from an elastic material such that the sleeve part (2) with the opening (10) can open and clipped onto the first part (1). On the outer wall surface (16), a plurality of rib shaped protrusions (17) protrude radially outward from the second part (2). In this embodiment, the ribs (17) are oriented parallel to the longitudinal axis of the sleeve shaped second part (2). Alternatively, the ribs are positioned at an angle with respect to this longitudinal axis.

The corresponding engagement members (9a, 9b) are shaped as a tooth, preferably as an asymmetric saw tooth. Each saw tooth comprises a saw cut surface (9c) and an undercut surface (9d) extending from the base (15) of the second part (2). The definition of the undercut surface and saw cut surface is shown and explained in Figure 1g.

Figure 1c presents the assembly of the first part (1) and the second part (2). In this embodiment, the second part (2) is clipped onto the first part (1). However as alternative, the first part (1) comprises two sub-units and the second part (2) is designed as a continuous ring without an opening. The first part and second part are assembled by inserting the second part (2) between the sub-units before assembling the sub-units of the first part (1). The Second part (2) can axially shift between two positions that are defined by the first flange (5) and second flange (6) of the first part. In Figure 1c, the second part (2) is positioned close to the second flange (6). Rotation of the first part (1) in the second rotation direction or clockwise direction (12) ensures that the undercut surface (4d) of the engagement member engages an undercut surface (9d) of a corresponding engagement member of the second part (2). In the example shown in figure 1c, the second part is shifted towards the second flange (6), but the engagement between the undercut surfaces also applies when the second part is shifted towards the first flange (5). Rotation of the first part (1) in the first rotation direction (11) ensures that saw cut surfaces of the engagement member (4c) and the corresponding engagement member (9c) can interact with each other for a mutual displacement engagement that will be explained below.

Figures 1d, 1e and 1f show the third part (3) of the first embodiment of the invention. Third part (3) is sleeve shaped comprising a cylindrical wall surface (18) which is closed by an end wall (19). The end wall comprises a coupling member (21) which protrudes outward form the end-wall (19) and is used to couple the third part to other units or parts of the injection or infusion device comprising the one-way coupling. A protrusion (20) protrudes inward from the end-wall (19) and is used for supporting the first part (1) or forming a bearing between the first part (1) and the third part (3). On the inside of the cylindrical wall surface (18), elements (22) protrude radially inward to form longitudinal grooves (23) between two neighboring elements (22). The grooves (23) extend axially parallel to the longitudinal axis of the sleeve shaped third part (3). Alternatively the grooves (23) are angulated with respect to the longitudinal axis of the third part. The longitudinal grooves (23) are designed to engage the radially extending ribs (17) of the second part (2).

Optionally the third part (3) comprises a circumferential groove (24) which is designed to engage a circumferential rim of a not shown housing or base of the injection or infusion device. As an alternative, the housing comprises a resilient member which snap fits into the groove (24) to ensure that the third part can rotate with respect to the housing while being axially fixed with respect to the housing.

Preferably there is a press-fit between the third part and the housing allowing for relative rotation between the third part (3) and the housing. The press fit can be established by the rim of the housing engaging the groove (24) or, alternatively a resilient member such as an O-ring is present between the third part and the housing. Preferably the press fit between the third part (3) and the housing forms a water tight and/or sterile barrier. The press-fit between the third part (3) and the housing is such that an initial frictional resistance, or threshold resistance needs to be over come before the third part starts rotating with respect to the housing. Alternatively there is a resilient element, for example an O-ring present in groove (24) which provides the friction fit engagement to the housing while providing a sterile and/or water tight barrier. To reduce friction, lubricants such as Teflon spray and/or silicon oil can be used between the third part (3) and the housing or between the third part and the resilient member.

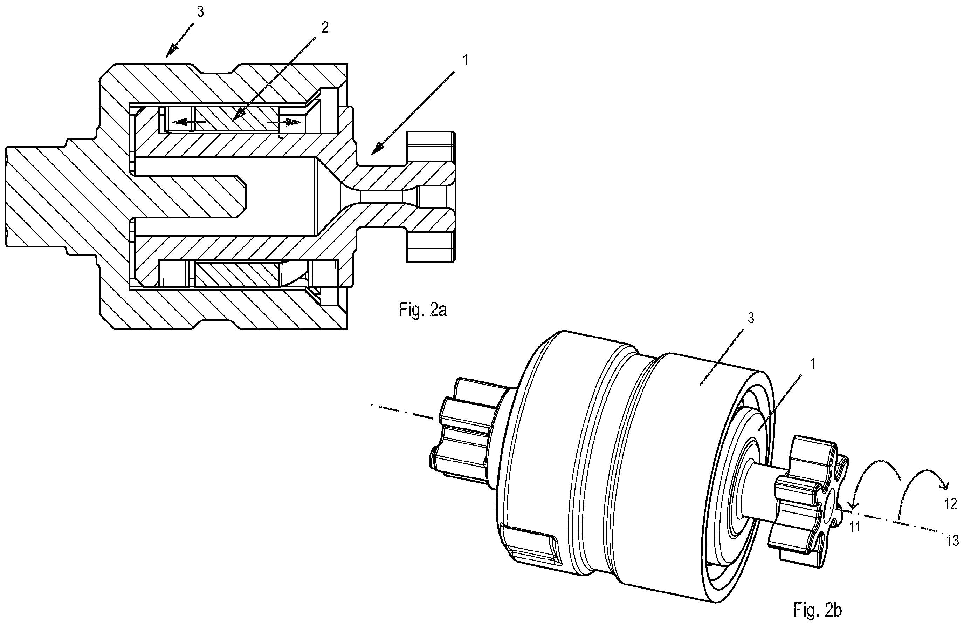

In Figures 2a and 2b, the assembled one-way coupling is presented comprising the first part (1), surrounded by the second part (2) which again is surrounded by the third part (3). The one-way coupling functions such that rotation and/or torque of the first part relative to the third part in the first rotation (11) direction is not transmitted to the third part whereas rotation of the first part in the second rotation direction (12) is transmitted to the third part. The rotation direction of the first part can be changed multiple times, each time when the rotation direction of the first part (1) relative to the third part (3) is in the first direction (11), rotation is not transmitted to the third part and each time the first part rotates in the second rotation direction (12) relative to the third part, ensures that the third part (3) rotates in the second rotation direction.

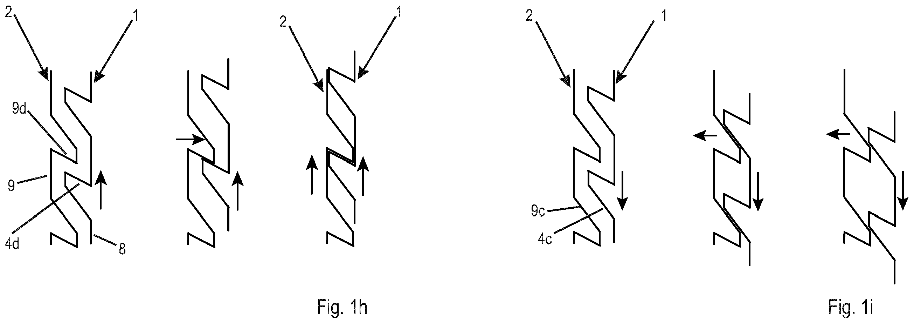

The one-way coupling is further explained using Figures 1g, Figure 1h and Figure 1i and Figure 2. Figure 1g shows a schematic representation of one engagement member or corresponding engagement member shaped as a saw tooth according to the first embodiment of the invention. The saw tooth protrudes from a base (8,15), having a saw cut surface (4c, 9c) and an undercut surface (4d, 9d). Both the saw cut surface as well as the undercut surface are angulated with respect to the base (8,15) with a first angle (25) and a second angle (26). The second angle (26) for the undercut surface (4d, 9d) is above the first angle (25) for the saw cut surface (4c, 9c), thus the undercut surface is steeper compared to the saw cut surface which is more shallow using this definition. The tip at the end of the undercut surface (27) of the saw tooth together with the undercut surface (4d, 9d) and the base (8,15) defines an undercut area between the base and the undercut surface. Optionally, the saw tooth can be designed with a top surface (28) that preferably extends parallel to the base (8,15) of the saw tooth and between the tip of the undercut surface (27) and the tip of the saw cut surface (29). The mutual locking engagement is schematically represented in Figure 1h. The first part (1) moves upward as indicated by the arrow and an engagement member, shown here as a saw tooth approaches a corresponding engagement member of the second part (2) that does not move. Once the undercut surfaces (4d, 9d) contact and slide along each other, the second part is forced to move towards the first part, indicated with the arrow oriented perpendicular to the first arrow. Once the top surfaces of the engagement member and the corresponding engagement member about the respective bases of the other part, then the first and second part are in a form fit or locking engagement and both the first and second part move together as indicated with the upward directed arrows. The mutual displacement engagement is shown in Figure 1i. The first part (1) moves in the downward direction as indicated with the arrow versus a non-moving second part (2). When the saw cut surface (4c) of the first part contacts and slides along the saw cut surface (9c) of the second part (2), then the second part is forced to move in a lateral direction due to the gearing arrangement between the two saw cut surfaces. For the proper functioning of the locking engagement and the displacement engagement it may be required that the first part rotates with respect to a third part, whereby the third part engages the second part. Preferably, the second part and/or the third part is in a friction fit engagement, for example to a housing or base of the device.

The functioning of the one-way coupling is explained using Figure 2a and Figure 1c. The first part (1) rotates in the first rotation direction (11) with respect to the third part (3). Preferably, the first part (1) and the third part (3) are axially fixed with respect to a housing or base of the device (not shown in the Figures). The engagement member (4a) in its rotary movement approaches a corresponding engagement member (9a) as the second part (2) is rotationally coupled to the third part (3) and thus the relative movement between the first part (1) and the third part (3) also applies for the relative rotational movement between the first part (1) and the second part (2). One of the pair of engagement members (4a, 4b) will contact one of the pair of corresponding engagement members (9a, 9b) due to the relative movement. The contact will be preferably in one of the two positions of the second part, in Figure 1c defined by the first flange (5) or second flange (6) that restrict the axial movement of the second part (2). A saw cut surface (4c) of an engagement member will slide over saw cut surface (9c) of a corresponding engagement member and the gearing engagement between the two surfaces will displace the second part either from the first position to the second position or from the second position to the first position. During further rotation of the first part, the other one of the pair of engagement members (4a,4b) which is opposite to the first engagement member, will contact the other one of the corresponding engagement members (9a,9b) such that the displacement engagement shifts the second part back to the initial position. This process is repeated and the second part (2) oscillates between the first and the second position as long as the first part rotates in the first rotation direction (11), as indicated by the arrows in Figure 2a. During the oscillating process, the second part (2), and therewith also the third part (3) do not rotate with respect to the rotating first part (1).

For the oscillation of the second part it is preferred that one of the pairs of engagement members or corresponding engagement members is positioned face-to-face to each other and the other one of the pairs of engagement members or the corresponding engagement members are rotationally displaced to another to ensure that the second part can shift back from one position to the other position.

When the first part (1) rotates in the second rotation direction (12) with respect to the third part (3), then one of the engagement members of a pair of engagement members (4a,4b) approaches one of the corresponding engagement members of a pair of corresponding engagement members (9a,9b). Upon contact of, the undercut surface (4d) of an engagement member (4a, 4b) comes in a mutual locking engagement with the undercut surface (9d) of a corresponding engagement member (9a, 9b) such that the second part enters in a form fit engagement or locking engagement with the first part (1) and torque is transmitted from the first (1) to the second part (2). Upon further rotation, the first part and the second part will co-rotate in the second rotation direction. In the first embodiment, the third part is in a rotational fixed engagement with the second part due to the ribs (17) engaging the longitudinal grooves (23) of the third part (3), and thus torque is transmitted from the first part via the second part to the third part. The third part (3) will co-rotate with the first part (1) upon further rotation of the first part in the second rotation direction (12).

The locking engagement between the first part (1) and the second part (2) is in one of the two axial positions that are available for the second part. Once locked, the second part (2) will remain in that position without any relative rotation or axial movement between the first part and the second part as long as the first part rotates in the second rotation direction.

As an alternative, the ribs (17) on the second part and the grooves (23) on the inside of the third part are off-axis to the longitudinal axis (13) defined by the first part. In this case a gearing engagement exists between the second part and the third part. Rotation of the first part in one of the two rotation directions will lead to an axial force that is applied on the third part via the gearing engagement. Preferably the third part has a degree of freedom for axial movement along the longitudinal axis such that the third part can axially move upon rotation of the first part relative to the third part.

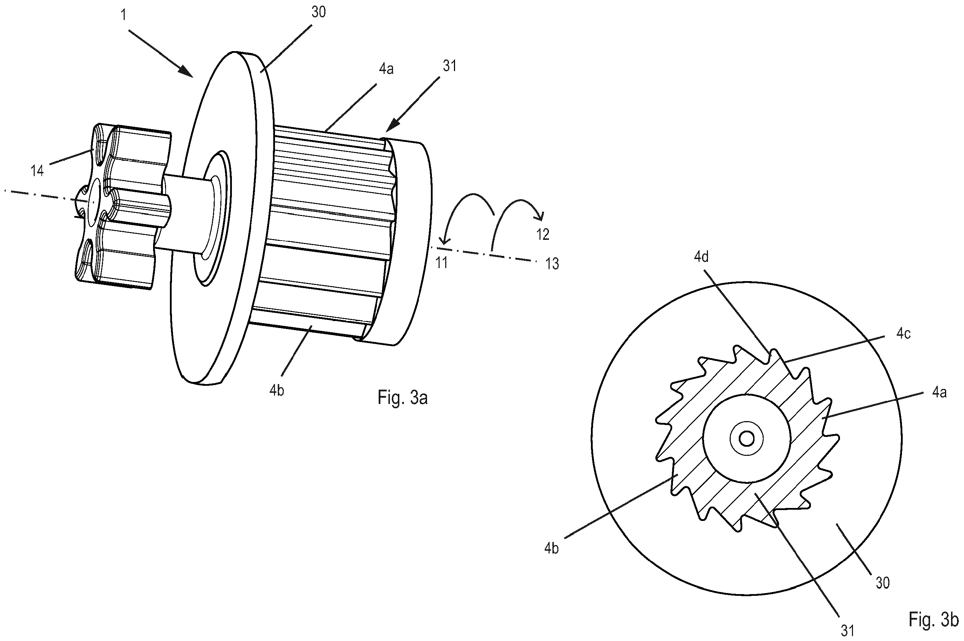

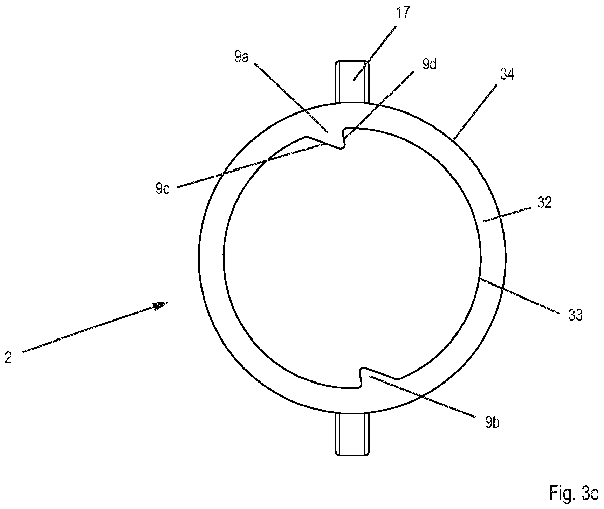

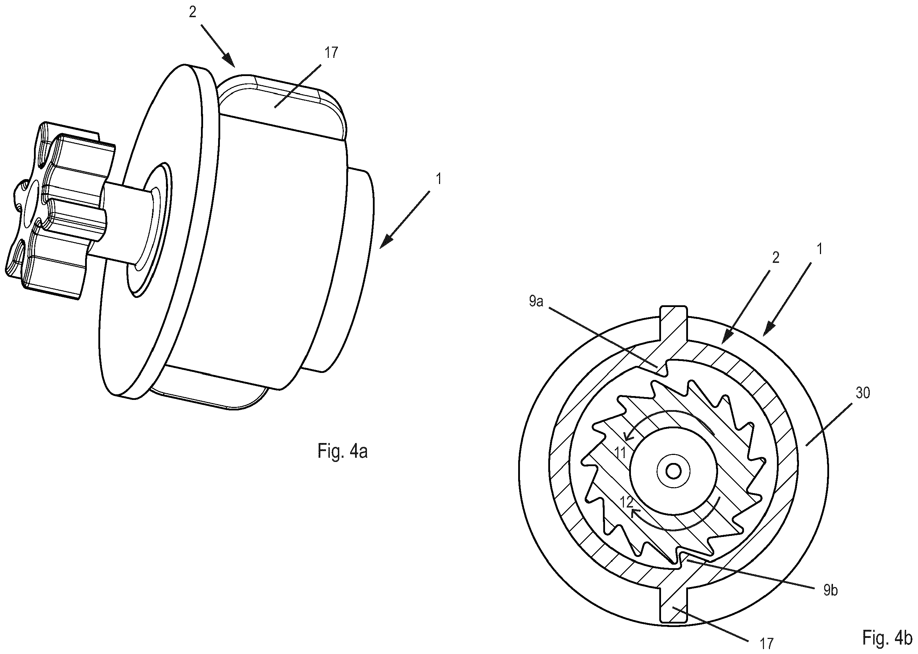

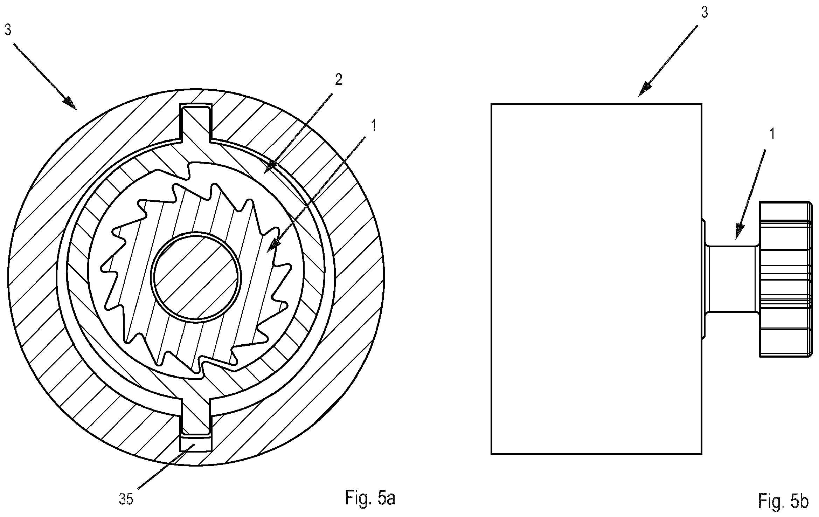

A one-way way coupling according to a second embodiment is shown in Figures 3 to 5. The first (1) comprises a gearwheel (14) which can be used for rotating the sleeve shaped first part (1) around its rotational axis (13) in the first rotation direction (11) or the second rotation direction (12), see Figure 3a. The gear wheel (14) is preferably attached to a restriction flange (30), the purpose of the flange is to close the one-way coupling and prevent excessive axial movement of the second part (2) along the rotational axis (13). The first part (1) comprises a sleeve (31) further comprising engagement members positioned on the outside surface of the sleeve (31) and which are shaped as asymmetric saw teeth. The saw teeth point radially outward, e.g. transversal to the longitudinal axis (13). The saw teeth extend parallel to the longitudinal axis (13). One of the engagement members (4a, 4b) is paired with an engagement member (4a, 4b) on the opposite side of the sleeve (Figure 3b). Each saw tooth comprises a saw cut surface (4c) and an undercut surface (4d), similar to the first embodiment. The second part (2), shown in Figure 3c, comprises a ring-shaped sleeve (32) with an inner diameter that fits around the sleeve (31) of the first part. On the inner surface (33) of the ring shaped sleeve (32), a pair of corresponding engagement members (9a, 9b) are positioned opposite to each other. Each of the corresponding engagement members of the pair of corresponding engagement members (9a, 9b) is shaped as an asymmetric saw tooth that points radially inward. The pair of corresponding engagement members are directed towards each other. Each saw tooth comprises a saw-cut surface (9c) and an undercut surface (9d). The tip of each saw tooth of the pair of corresponding engagement members (9a, 9b) is directed in a circumferential direction opposite to the direction of the tip of each saw tooth of the engagement members of the first part. In the example presented in Figures 3b and 3c, the tips of the saw teeth of the engagement members are directed in the counterclockwise direction whereas the tips of the saw teeth of the corresponding engagement members point in the clockwise direction. The saw teeth of the engagement members and corresponding engagement members preferably have complementary or mirrored shapes. The outside surface (34) of the ring-shaped sleeve (32) comprises two axially extending ribs (17) (Figure 4a) that are positioned opposite to each other and preferably at the same location on the circumference of the ring-shaped sleeve(32) as the corresponding engagement members (9a,9b). The third part (3) is a sleeve that surrounds the second part (2) and is equipped with at least two grooves (35) that engage the ribs (17) of the second part (2) such that the second part (2) and third part (3) can move axially relative to another while being in a rotationally locked engagement. Alternatively, the position of the ribs and the grooves on the second and third part is reversed. Preferably, the third part is in engagement with the housing or base of the injection or infusion device. Preferably the first part and the third part are restricted or limited in the axial movement with respect to the housing.

The one-way coupling according to the second embodiment functions as follows. Rotation of the first part with respect to the third part (3) in the first rotation direction (11) brings one of the engagement members (4a, 4b) in a locking engagement such that torque is transmitted from the first part (1) to the second part (2). The locking engagement is triggered by the undercut surfaces (4d, 9d) that engage each other as described previously. Since the second part is rotationally secured to the third part, also torque is transmitted to the third part. Upon further rotation of the first part, all three parts will co-rotate in the first rotation direction. Rotation of the first part in the second rotation direction (12) which is opposite to the first rotation direction, brings one of the saw teeth (4a,4b) of the first part in a mutual displacement engagement with one of the saw teeth (9a, 9b) of the second part. The saw cut surfaces (4c, 9c) of the engagement member and corresponding engagement member slide over each other and displaces the second part from one position to the other position and the second part starts oscillating transversally to the longitudinal axis without rotation. The rotation of the first part is therefore also not transmitted to the third part.

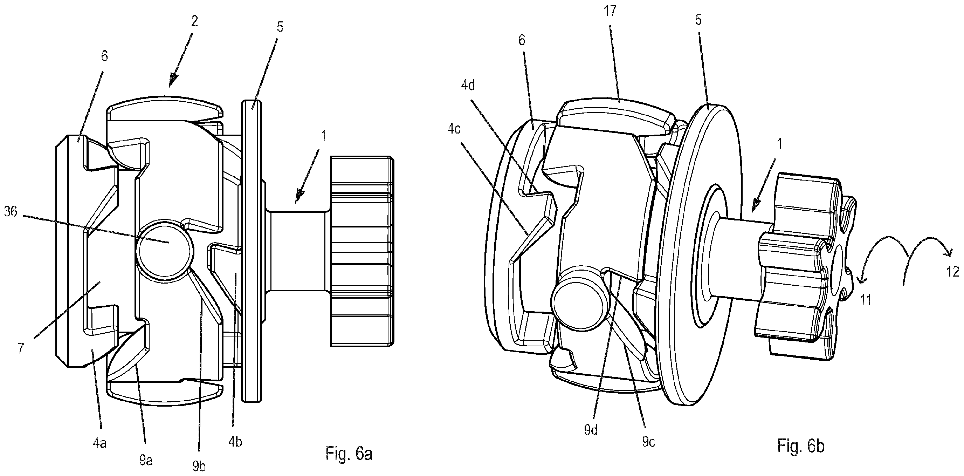

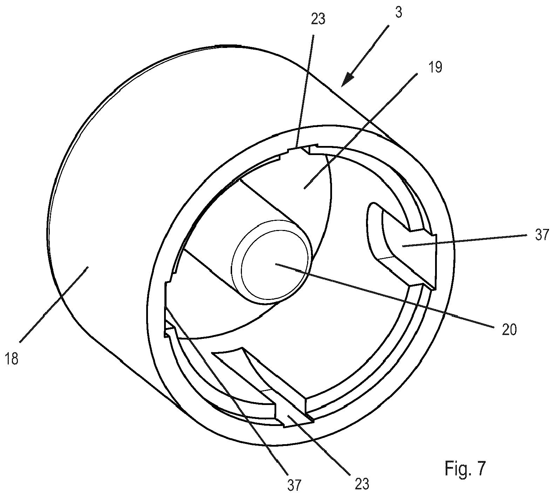

A one-way coupling according to a third embodiment is shown in Figures 6 to 8. The first part (1) resembled the first part of the first embodiment. The first part (1) comprises a sleeve shaped body (7) with two radially extending flanges (5, 6) at each end of the body (7). The engagement members (4a, 4b) are attached to the flanges (5,6) and are shaped as saw teeth that point towards each other. Different from the first embodiment, the engagement members of each pair of engagement members are off-set to each, positioned at a different angle on the circumference of the flanges (5, 6). The second part (2) is shaped as a ring surrounding the body (7) of the first part. The second part (2) is coupled to the first part (1) via a hinge (36) such that the second part can pivot with respect to the first part (1). The second part (2) has a plurality of corresponding engagement members (9a, 9b) positioned on the circumference of the ring. Pairs of corresponding engagement members (9a, 9b) are positioned off-set to each other. The corresponding engagement members (9a, 9b) are designed as cut-outs of the ring shaped second part (2). Both the engagement members as well as the corresponding engagement members have sloped surfaces that act either as a saw cut surface (4c, 9c) or as an undercut surface (4d, 9d). At least one protrusion (17) is pointing radially outward from the second part. In this embodiment, the protrusion is shaped as a longitudinal rib oriented parallel to the longitudinal axis of the second part (2). The third part (3) (Figure 7) is designed as sleeve with a cylinder wall surface (18) an end wall (19) from which a protrusion (20) protrudes. On the inner surface of the cylinder wall (18) longitudinal grooves (23) extend parallel to the longitudinal axis of the sleeve which are designed to engage the ribs (17) of the second part. Furthermore recessed sections (37) are present on the inner wall surface for receiving a part of the hinge (36). The assembled one-way coupling is shown in Figures 8a and 8b. The sleeve shaped first part (1) is axially supported by the protrusion (20) of the third part. Upon rotation of the first part (1) in the first rotation direction (11), the saw teeth (4a,4b) engage with the cut-outs (9a,9b) in a mutual displacement engagement which ensures that the second part is pivoted around the hinge (36) between a first and a second position. The first and second positions for the second part are shown in Figures 8a and 8b, respectively. The second part (2) is tilted without rotation when the first part is rotated in the first rotation direction and no torque or rotation is transferred to the third part (3). Rotation of the first part in the second rotation direction (12) brings the engagement members and corresponding engagement members in a mutual locking configuration such that the second part is either in the first position or second position (Figure 8a or Figure 8b) and torque is transmitted from the first part to the second part. Finally the first, second and third part co-rotate in the second rotation direction.

A one-way coupling according to a fourth embodiment is shown in Figure 9. The second part (2) is sleeve shaped and surrounds the first part (1). The axial displacement of the second part (2) is restricted by flanges (5, 6) that radially extend from the body of the first part. The engagement member (4a) on the second flange (6) is identical to the first embodiment and comprises asymmetrical saw-teeth with a saw cut and an undercut surface. The engagement member (4e) on the first flange (5) of the first part is shaped as a symmetric tooth e.g. both flanks of the tooth have an equal slope with respect to the base. A pair of engagement members (4a, 4e) of the first part thus comprises one symmetric and one asymmetric tooth. The second part (2) is sleeve shaped having mating corresponding engagement members (9a, 9e). The corresponding engagement member closest to the first flange (5) of the first part comprises a symmetric tooth (9e). Rotation of the first part in the first rotation direction (11) brings the engagement members and corresponding engagement members of the first and second part in a mutual displacement engagement and the second part shifts back and forth without transmitting any torque via ridges (17) to a not shown third part (3). Rotation of the first part in the second rotation direction (12) brings the engagement member (4a) and corresponding engagement member (9a) in a mutual locking engagement and the second part co-rotates with the first part. If the second part is however on the opposite side, closest to the first flange (5), then rotation of the first part in the second rotation direction first shifts the second part (2) towards the second flange (6) since the symmetric teeth provide a mutual displacement engagement for both rotation directions. With this configuration of the one-way coupling the second part is always in one of the two positions and can be selected by designing the shape of the engagement members and/or corresponding engagement members. The advantage of having the second part, like in this example, in the position closest to the second flange may be to support an additional functionality such as providing an electrical contact between the second flange (6) and the second part (2) and avoiding an electrical contact between the first flange (5) and the second part (2). Thus by steering the position of the second part, an additional functionality can be combined with the one-way coupling. A further advantage may be that the teeth that always engage are made from a stiffer or high strength material whereas the teeth on the opposite side are made from a more cost efficient material.

A one-way coupling according to a fifth embodiment is shown in Figure 10. The fifth embodiment represent a combination between the first and the third embodiment in that the second part (2) performs a combination of axial movement and tilting movement while moving back and forth between the first and the second position. The second part (2) is positioned between the flanges (5, 6) of the first part. The engagement members (4a) has an additional sloped surface (4f) that starts at the flange (4) and goes down to the body (7) of the first part. The corresponding engagement members have mating sloped surfaces (9f) such that the displacement engagement ensures that the second part axially moves and tilts while going from one position to the other position. The advantage compared to the third embodiment is that no hinge is present between the first and the second part. The functioning of the one-way coupling and the torque transfer from the second part to the third part via protrusions (17) is identical to the first and third embodiment.

A one way coupling according to a sixth embodiment is shown in Figure 11. The sixth embodiment resembles the second embodiment in that the second part (2) moves transversally while oscillating between the two positions. The first part comprises the engagement members (4a, 4b) but there are four sets of saw teeth that are axially spaced along the longitudinal axis. Each set of saw teeth s rotationally displaced with respect to another set of saw teeth. For each set of saw teeth, a ring shaped second part is used having corresponding engagement members for interacting with the engagement members of the first part. In total four ring shaped second parts (2) are shown in Figure 11. Rotation of the first part in the first rotation direction will set the engagement members in a mutual displacement engagement with the four ring shaped second parts. The four ring shaped parts will axially move perpendicular to the rotational axis of the first part (1). Rotation of the first part in the second rotation direction ensures that one of the four second parts comes as a first in a locking engagement and transfers the torque from the first part to the second part. The advantage of having more than one second part is that the rotational play before rotation is transferred from the first part to the second part is reduced. In the sixth embodiment the torque is transmitted from the second part to the third part via a form fit engagement and not using the ribs of the second embodiment.

| | |
|---|---|
| **Part annotation** | (13) Rotation axis first part |
| (1) First part | (14) Gearwheel |
| (2) Second part | (15) Second base |
| (3) Third part | (16) Outer wall surface |
| (4a, 4b) Pair of two engagement members | (17) Rib, protrusion |
| (4c) Saw cut surface engagement member | (18) Wall surface |
| (4d) Undercut surface engagement member | (19) End wall |
| (4e) Symmetric engagement member | (20) Protrusion for bearing |
| (4f) Sloped surface | (21) Coupling member |
| (5) First flange | (22) Element |
| (6) Second flange | (23) Longitudinal groove |
| (7) Cylindrical body or sleeve | (24) Circumferential groove |
| (8) Base | (25) First angle |
| (9a, 9b) Pair of two corresponding engagement members | (26) Second angle |
| | (27) Tip of undercut surface |
| (9c) Saw cut surface corresponding engagement member | (28) Top surface of saw tooth |
| | (29) Tip of saw cut surface |
| (9d) Undercut surface corresponding engagement member | (30) Restriction flange |
| | (31) Sleeve first part |
| (9e) Symmetric corresponding engagement member | (32) Ring shaped sleeve second part |
| | (33) Inner surface |
| (9f) Sloped surface | (34) Outer surface |
| (10) Opening | (35) Groove |
| (11) First rotation direction | (36) Hinge |
| (12) Second rotation direction | (37) Recessed section |

## Claims

1. A one-way coupling for an injection device or an infusion device, the one-way coupling comprising:
a first part (1) defining a longitudinal axis (13) and being restricted for movement along and rotatable around the longitudinal axis, the first part having at least one pair of two engagement members (4a, 4b) which are positioned spaced apart from each other,
at least one second part (2) coaxially arranged with respect to the first part (1) and having at least one pair of two corresponding engagement members (9a, 9b) configured for engaging the at least one pair of two engagement members (4a, 4b) of the first part (1), the second part (2) being moveable with respect to the first part (1) between a first and a second position,
a third part (3) coaxially arranged with respect to the first (1) and second part (2), the third part being restricted for movement along the longitudinal axis and configured for engaging the second part (2) such that the second part (2) is rotationally secured with respect to the third part (3),
**characterized by**
that rotation of the first part (1) around the longitudinal axis (13) in a first rotation direction (11) relative to the third part (3) brings one engagement member of the at least one pair of two engagement members (4a,4b) in a mutual displacement engagement with one corresponding engagement member of the at least one pair of two corresponding engagement members (9a, 9b), thereby displacing the second part (2) from the first position to the second position or from the second position to the first position such that the second part moves back and forth between the first and second position,
wherein rotation of the first part (1) around the longitudinal axis (13) in a second rotation direction (12) relative to the third part (3), the rotation of the first part being opposite to the first rotation direction (11), brings one engagement member of the at least one pair of two engagement members (4a, 4b) in a mutual locking engagement with one corresponding engagement member of the at least one pair of two corresponding engagement members (9a, 9b) such that the first, second and third part are coupled in a form-fit engagement for rotational movement in the second rotation direction and/or respective torque transmission.

2. The one-way coupling according to claim 1, wherein the two engagement members of each pair of the at least one pair of two engagement members (4a, 4b) of the first part (1) are spaced apart and oppositely directed and parallel or transversally aligned with respect to the longitudinal axis (13),

3. The one-way coupling according to claims 1 or 2, wherein the at least one pair of two corresponding engagement members (9a, 9b) of the second part (2) are oppositely directed and arranged between the at least one pair of two engagement (4a, 4b) of the first part,

4. The one-way coupling according to claims 1 to 3 wherein the first and second position of the second part (2) is defined by the position of the two engagement members of the at least one pair of two engagement members (4a, 4b) of the first part (1),

5. The one-way coupling according to claims 1 to 4 wherein the second part (2) moves back and forth between the first and second position such that this movement is parallel, perpendicular or tilted with respect to the longitudinal axis (13),

6. The one-way coupling according to any of the previous claims wherein the first, second and third part are sleeve shaped or shaft shaped,

7. The one way coupling according to any of the previous claims wherein the second part is located between the first and third part, or the second and third part are surrounded by the first part,

8. The one-way coupling according to any of the previous claims wherein the second part (2) is axially moveable and rotationally secured with respect to the third part (3),

9. The one-way coupling according to any of the previous claims wherein the second part (2) moves back and forth between the first and second position without rotation when the first part (1) rotates relative to the third part (3) in the first rotation direction (11),

10. The one-way coupling according to any of the previous claims wherein the second part (2) has an engagement means for rotationally locking to the third part (3), preferably designed as a radial protrusion (17) on the second part (2) that engages a radial recession (23, 35) on the third part (3), or designed as a radial recession on the second part (2) that engages a radial protrusion on the third part (3), wherein the engagement means allows for relative axial or pivoting movement between the second (2) and third part (3),

11. The one way coupling according to any of the previous claims wherein the third part (3) is rotationally engaged in a friction fit arrangement to a housing or base of the injection or infusion device,

12. The one-way coupling according to any of the previous claims wherein the first part (1) is axially supported by a bearing engagement between the first part (1) and the third part (3),

13. The one-way coupling according to any of the previous claims wherein the second part (2) is a sleeve having a longitudinal opening (10) to form a C-arc that can be clipped onto the first part (1) or wherein the first part (1) is constructed as two sub-parts that can be assembled together after insertion of the second part (2),

14. The one-way coupling according to any of the previous claims wherein the at least one pair of two engagement members (4a, 4b) are circumferentially arranged on the first part (1) around the longitudinal axis (13) and the at least one pair of two corresponding engagement members (9a,9b) are circumferentially arranged on the second part (2), whereby the two engagement members (4a, 4b) of the at least one pair of two engagement members of the first part are positioned face-to-face to each other and the two corresponding engagement members (9a, 9b) of the at least one pair of two corresponding engagement members are positioned off-set to each other, or the two engagement members (4a, 4b) of the at least one pair of two engagement members of the first part (1) are positioned off set to each other and the two corresponding engagement members of the at least one pair of two corresponding engagement members (9a, 9b) of the second part (2) are positioned face-to-face to each other,

15. The one-way coupling according to any of the previous claims wherein the engagement members and corresponding engagement members are shaped as teeth, preferably saw-teeth,

16. The one-way coupling according to claim 15 wherein the mutual displacement arrangement and/or the mutual locking engagement is caused by sloped surfaces (4c, 4d, 4f, 9c, 9d, 9f) of the teeth of the engagement members and the corresponding engagement members sliding along each other,

17. The one way coupling according to claims 15 or 16 wherein the engagement members of the first part protrude from a base (8,15) present on the first part (1),

18. The one-way coupling according to claim 17 wherein the second part (2) is present in a recessed section of the first part (1) or between two flanges (5,6, 30) present on the first part (1), wherein the base (8,15) for the engagement members (4a, 4b) is formed by ridges of the recessed section of the first part, or by the flanges (8,15) present on the first part (1),

19. The one way coupling according to claims 15 or 18 wherein the teething comprises saw teeth, and wherein the locking engagement is defined by the mutual interaction of the undercut surface (4d) of a saw tooth from an engagement member (4a, 4b) with an undercut surface (9d) of a saw tooth from a corresponding engagement member (9a, 9b) causing a form fit arrangement between the engagement member and the corresponding engagement member when the two undercut surfaces (4d, 9d) slide along each other, and wherein the displacement engagement is defined by the mutual interaction of a saw-cut surface (4c) from an engagement member (4a, 4b) with a saw cut surface (9c) from a corresponding engagement member (9a, 9b) causing a gearing arrangement between the engagement member and the corresponding engagement member when the two saw cut surfaces (4c, 9c) slide along each other causing relative axial displacement of the engagement member and corresponding engagement member,

20. Injection or infusion device comprising the one way coupling of any of the previous claims, configured for coupling and decoupling the rotational movement of an electromotor to a drive mechanism or a needle insertion mechanism by switching the rotation direction of the first part (1) from the first direction (11) to the second rotation direction (12).
